# EUROPÄISCHE PATENTSCHRIFT

(11) **EP 0 319 854 B1**
(45) Veröffentlichungstag und Bekanntmachung des Hinweises auf die Patenterteilung: **02.03.1994**
(21) Anmeldenummer: 88120139.6
(22) Anmeldetag: 02.12.1988
(51) Int. Cl.: C07D 473/06, C07D 473/08

(54) **Verfahren zum Methylieren von Xanthinen**
Process for the methylation of xanthins
Procédé pour la méthylation de xanthines

(30) Priorität: 10.12.1987 DE 3741883
(43) Veröffentlichungstag der Anmeldung: 14.06.1989
(73) Patentinhaber: BOEHRINGER INGELHEIM KG, 55216 Ingelheim (DE); BOEHRINGER INGELHEIM INTERNATIONAL GmbH, 55218 Ingelheim am Rhein (DE)
(72) Erfinder: Hilgers, Georg, Dr., D-6507 Ingelheim/Rhein (DE); Kröber, Jutta, D-6530 Bingen-Dietersheim (DE)

(56) Entgegenhaltungen:
- LIEBIGS ANNALEN DER CHEMIE, Nr. 1, Januar 1987, Seiten 77-79, Weinheim, DE; M. LISSEL: "N-Methylierung von Imidazol und Derivaten"

## Beschreibung

Die Erfindung betrifft ein Verfahren zum Methylieren von Xanthinen unter Verwendung von Dimethylcarbonat als Methylierungsmittel. Unter dem Ausdruck Xanthine werden Xanthine der allgemeinen Formel 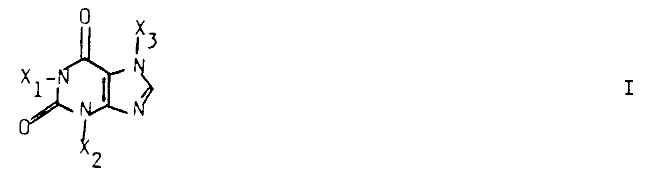
sowie deren Alkali- und Erdalkalisalze, insbesondere Natrium-, Kalium- und Calciumsalze zusammengefaßt, worin X₁, X₂ und X₃ unabhängig von einander Wasserstoff, C₁-C₈-Alkyl, Phenyl oder Benzyl bedeuten, wobei wenigstens einer der Substituenten X₁, X₂ und X₃ Wasserstoff bedeutet.

Die so hergestellten Verbindungen haben physiologische Aktivität und/oder sind als Ausgangsmaterial für die Herstellung anderer physiologisch wirksamer Verbindungen (z.B. Pentifyllin, Pentoxifyllin, Protheobromin) von Bedeutung.

Es ist bekannt, Theobromin aus Xanthin oder 3-Methylxanthin sowie Coffein aus Theophyllin durch Methylieren mit Dimethylsulfat, Methylchlorid oder Methyltosylat herzustellen. Diese Methylierungsreagentien sind gesundheitsschädlich und ergeben gesundheitsschädliche Nebenprodukte bei der Umsetzung. Ferner befriedigen Ausbeute und Produktqualität dieser Verfahren zur Herstellung von insbesondere Theobromin nicht.

Dimethylcarbonat ist als Methylierungsmittel bekannt, das wesentlich schwächer wirkt als Dimethylsulfat, Dimethylcarbonat ist nicht gesundheitsschädlich und führt auch nicht zu gesundheitsschädlichen Nebenprodukten. (Chimicaoggi - ottobre 1983, 33-34; Chimicaoggi - setembre 1984, 37-45; Ing. Chim. Ital., V. 21, N. 1-3, Gen.-Mar. 1985, 6-12; Liebigs Ann. Chem. 1987, 77-79).

In Liebigs Ann. Chem. 1987, 77-79 wird ein Methylierungsverfahren beschrieben, in dem Imidazol, substituierte Imidazole und Indol mit Dimethylcarbonat methyliert werden. Nach dieser Beschreibung werden die gewünschten Produkte mit befriedigender oder guter Ausbeute erhalten, wenn geeignete Katalysatoren verwendet werden. Erwähnt wird darin auch die Methylierung von Imidazol ohne Verwendung eines Katalysators, allerdings ist dabei für eine Ausbeute von 90 % eine Reaktionszeit von 72 Stunden angegeben. Dieses Verfahren ist für technische Anwendungen nicht geeignet, insbesondere wegen der langen Reaktionszeit, wenn auf Katalysatoren verzichtet wird, und wegen der aufwendigen Isolierung der Endprodukte, wenn das Verfahren mit den angegebenen Katalysatoren ausgeführt wird. Versuche zeigten ferner, daß nach diesem Verfahren die Methylierung von Xanthinen nicht oder nur mit sehr geringen Ausbeuten möglich ist.

Aufgabe war es, ein verbessertes, technisch anwendbares Verfahren zu entwickeln, bei dem keine gesundheitsschädlichen Methylierungsmittel verwendet werden.

Durch die vorliegende Erfindung wird ein Verfahren bereitgestellt, das methylsubstituierte Xanthine in hoher Ausbeute und Qualität ergibt. Das Verfahren ist auch in technischem Maßstab einfach auszuführen, insbesondere da es nur wenige Schritte für die Isolierung des Produktes benötigt. In dem erfindungsgemäßen Verfahren wird kein Katalysator und keine Base verwendet. Es ist neu, Dimethylcarbonat auf bicyclische Heterocyclen anzuwenden, die in beiden fusionierten Ringen je zwei Stickstoffatome enthalten.

Gemäß der Erfindung werden Xanthine unter Verwendung von Dimethylcarbonat methyliert. Die Substanzen werden in einem Molverhältnis jeweiliges Xanthin (nach obiger Definition) zu Dimethylcarbonat gleich 1 : 3 bis 1 : 50, vorzugsweise 1 : 7 bis 1 : 10 eingesetzt. Dimethylcarbonat dient gleichzeitig als Lösungsmittel. Die Umsetzung wird vorzugsweise mit Zusatz von Wasser oder einem wässerigen Puffer (ausgelegt für pH 7,8 bis 7,1) oder von organischem Lösungsmittel mit acidem Wasserstoff, z.B. einem Alkohol, insbesondere C₁-C₅ Alkohol, vorzugsweise Methanol ausgeführt.

Die Umsetzung wird bei 100 bis 250°C, vorzugsweise 150 (insbesondere 170) bis 200°C in einem Autoklaven unter entsprechend erhöhtem Druck (1-160 bar, meist 50-80 bar) durchgeführt. Die Dauer der Umsetzung liegt meist zwischen einer und 10 Stunden.

Bedingt durch die verschiedene Reaktivität der in den Ausgangsverbindungen enthaltenen Stickstoffatome erfolgt die Methylierung nach dem erfindungsgemäßen Verfahren schrittweise. Durch die Wahl der Reaktionsbedingungen und Reaktionsdauer innerhalb der angegebenen Bereiche kann die Ausbeute an den einzelnen Endverbindungen optimiert werden. Als Nebenprodukte treten unerwünschte Methylierungsprodukte auf. Bei der Herstellung von Theobromin fallen z.B. geringe Mengen von Coffein und 3-Methylxanthin (bei der Verwendung von Xanthin) als Nebenprodukte an.

Die Ausgangsverbindungen sind bekannte Verbindungen und/oder können nach an sich bekannten Verfahren hergestellt werden.

Vorzugsweise wird das erfindungsgemäße Verfahren für die Herstellung von Theobromin aus Xanthin oder 3-Methylxanthin und für die Herstellung von Coffein aus Theophyllin.,Xanthin oder 3-Methylxanthin verwendet.

Für die Herstellung von Theobromin werden dabei Xanthin bzw. 3-Methylxanthin und Dimethylcarbonat in einem Molverhältnis von 1 : 3 bis 1 : 50, vorzugsweise 1 : 7 bis 1:10, eingesetzt. Ferner wird vorzugsweise Wasser oder ein wäßriger Puffer für pH 7,8-7,1 zugesetzt. Dabei werden vorzugsweise gleiche Volumsteile Wasser bzw. Puffer eingesetzt wie Dimethylcarbonat. Die Reaktionstemperatur wird auf 100 bis 250°C, vorzugsweise 170 bis 200°C, gehalten; die Reaktion wird gewöhnlich nach 2,5 Stunden beendet, vorzugsweise indem auf etwa 70°C abgekühlt wird und der Autoklav belüftet wird. Die Aufarbeitung besteht aus Abtrennen des Reaktionsproduktes (Filtrieren, Absaugen), Waschen und Trocknen.

Für die Herstellung von Coffein wird vorzugsweise Xanthin, 3-Methylxanthin oder insbesondere Theophyllin oder deren Na- oder Ca-Derivate mit Dimethylcarbonat in einem Molverhältnis von 1 : 3 bis 1 : 50, vorzugsweise 1 : 7 bis 1 : 10 eingesetzt. Es ist vorteilhaft, einen Zusatz von Alkohol, vorzugsweise Methanol bei der Umsetzung zu verwenden. Dabei beträgt das Volumsverhältnis Dimethylcarbonat zu Methanol 3,3 zu 1 bis 2 zu 1. Die Umsetzung erfolgt im Autoklaven bei 100 bis 250°C, vorzugsweise 150 bis 200°C. Nach einer Reaktionsdauer von 1 bis 10 Stunden, vorzugsweise 3 bis 5 Stunden, wird die Reaktion durch Abkühlen auf etwa 60°C und Entlüften des Autoklaven beendet. Die Aufarbeitung der Reaktionsmischung erfolgt durch Abtrennen des Reaktionsproduktes (Filtrieren, Einengen des Filtrats, Ausrühren im Eisbad), Waschen und Trocknen.

Nach diesem Verfahren können ebenso z.B. 3-(C₁-C₈-alkyl)-Xanthin, 3-Phenylxanthin oder 3-Benzylxanthin umgesetzt werden. Aus so hergestelltem 3-Benzyl-7-methyl-xanthin kann durch Abspalten der Benzylgruppe unter Verwendung herkömmlicher Methoden (z.B. Hydrierung) 7-Methylxanthin hergestellt werden.

### Beispiel 1

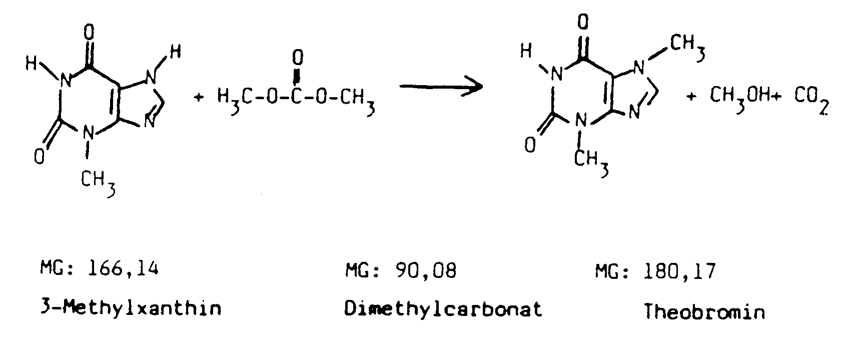

In einem 2 l Schüttelautoklav werden 500 ml Puffer pH 7,5 (hergestellt aus 13,68 g Na₂B₄O₇ x 4 H₂O und 94 ml 1 N Salzsäure aufgefüllt auf 1 l) und 500 ml Dimethylcarbonat (6 Mol) vorgelegt. Dazu gibt man 99,6 g (0,6 Mol) 3-Methylxanthin und erhitzt die Suspension auf 170°C. Während des 2 1/2 stündigen Schüttelns steigt der Druck auf bis zu 100 bar an. Nach Ende der Reaktionszeit wird auf 70°C abgekühlt und der Autoklav belüftet. Anschließend saugt man die Suspension (pH∼7,2) über eine vorgeheizte Nutsche mit Filterpapier heiß ab und wäscht mit 2 x 100 ml Wasser nach. Die isolierte Substanz wird bei 50°C im Vakuumtrockenschrank bis zur Gewichtskonstanz getrocknet.

| | | | | | |
|---|---|---|---|---|---|
| HPLC | 99,8 % | | | | |
| C,H,N | Theorie: | C | 46,66 % | gef.: | 46,62 % |
| | | H | 4,48 % | | 4,48 % |
| | | N | 31,10 % | | 31,03 % |

Schmelzpunkt: 352-355°C
Ausbeute: 79,1 g (≙ 73,23 %)
Analog zu diesem Beispiel kann 3-Methylxanthin-Na zu Theobromin mit einer Ausbeute von 79 % umgesetzt werden. (Reaktionsdauer 2,5 Stunden).

### Beispiel 2

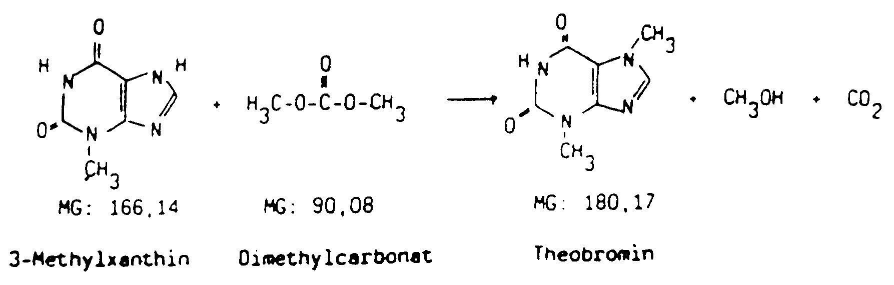

In einem 2 l Schüttelautoklav werden 500 ml Wasser und 500 ml Dimethylcarbonat vorgelegt. Dazu gibt man 99,6 g (0,6 Mol) 3-Methylxanthin und erhitzt die Suspension auf 175°C. Während des 2 1/2 stündigen Schüttelns steigt der Druck auf 80 bar an. Nach Ende der Reaktionszeit wird auf 70°C abgekühlt und der Autoklav belüftet. Anschließend saugt man die Suspension(pH∼ 7,1) über eine vorgeheizte Nutsche mit Filterpapier heiß ab und wäscht mit 2 x 100 ml Wasser nach. Die isolierte Substanz wird bei 50°C im Vakuumschrank bis zur Gewichtskonstanz getrocknet.

| | | | | | |
|---|---|---|---|---|---|
| C,H,N | Theorie: | C | 46,66 % | gef.: | 46,53 % |
| | | H | 4,48 % | | 4,47 % |
| | | N | 31,10 % | | 30,77 % |
| HPLC | 98,6 % | | | | |

Schmelzpunkt: 351-355%
Ausbeute: 71,17 g (≙ 65,84 %)

### Beispiel 3

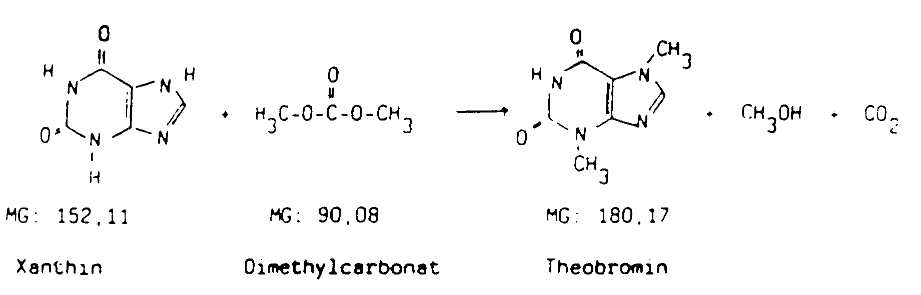

In einem 2 l Schüttelautoklav werden 500 ml Puffer pH 7,8 (hergestellt aus 6,84 g Na₂B₄O₇ x 4 H₂O und 41 ml 1 N Salzsäure aufgefüllt auf 500 ml) und 500 ml Dimethylcarbonat vorgelegt. Dazu gibt man 91,27 g (0,6 Mol) Xanthin und erhitzt die Suspension auf 200°C. Während des 2 1/2 stündigen Schüttelns steigt der Druck auf 160 bar an. Nach Ende der Reaktionszeit wird auf 70°C abgekühlt und der Autoklav belüftet. Anschließend saugt man die Suspension (pH∼7,6) über eine vorgeheizte Nutsche mit Filterpapier heiß ab und wäscht mit 2 x 100 ml Wasser nach. Die isolierte Substanz wird bei 50°C im Vakuumtrockenschrank bis zur Gewichtskonstanz getrocknet.

| | | | | | |
|---|---|---|---|---|---|
| C,H,N | Theorie: | C | 46,66 % | gef.: | 46,50 % |
| | | H | 4,48 % | | 4,59 % |
| | | N | 31,10 % | | 30,70 % |
| HPLC | 98,5 % | | | | |

Schmelzpunkt: 351-354°C
Ausbeute: 45,61 g (≙ 42,19 %)

### Beispiel 4

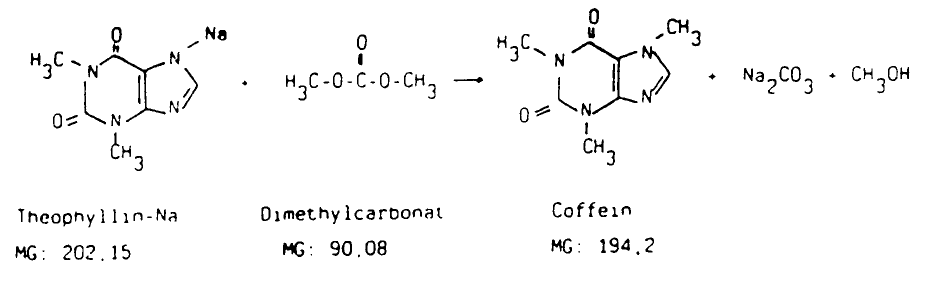

In einem 1 l Schüttelautoklav werden 100 ml Methanol, 334 ml Dimethylcarbonat und 90 g (0,4 Mol) 90 %-iges Theophyllin-Natriumsalz vorgelegt. Die Suspension erhitzt man auf 200°C und schüttelt 3 Std. bei dieser Temperatur. Hierbei steigt der Druck bis auf ca. 40 bar an. Nach Ende der Reaktionszeit wird auf ca. 60°C abgekühlt, belüftet und die Suspension über eine vorgeheizte Nutsche mit Filterpapier heiß abgesaugt. Der Filterrückstand (Natriumcarbonat) wird mit Methanol nachgewaschen. Von den vereinigten Lösungen werden 400 ml Methanol abdestilliert. Dann wird die erhaltene Suspension 1 Std. im Eisbad gerührt. Das ausgefallene Coffein wird abgesaugt, mit kaltem Methanol gewaschen und bei 60°C im Vakuumtrockenschrank bis zur Gewichtskonstanz getrocknet. Zur Gewinnung von weiterem Produkt wird von den vereinigten Laugen Methanol über eine Kolonne bis zu einem Restvolumen von 100 ml abdestilliert. Die hierbei anfallende Suspension wird 1 Std. im Eisbad gerührt, abgesaugt und mit kaltem Methanol nachgewaschen. Die Zweitkristalle werden gemeinsam mit den Erstkristallen getrocknet.

| | | | | | |
|---|---|---|---|---|---|
| Ausbeute | 69,9 g (≙ 90 %) | | | | |
| Fp | 234-236°C | | | | |
| HPLC | 99,7 % | | | | |
| C,H,N | Theorie: | C: | 49,48 % | gef.: | 49,34 % |
| | | H: | 5,19 % | | 5,20 % |
| | | N: | 28,85 % | | 28,74 % |

Bei Einsatz von Theophyllin oder Theophyllin-Ca als Ausgangsmaterial wird analog verfahren. Bei der Verwendung von Theophyllin ist eine Reaktionsdauer von vorzugsweise bis zu 5 Stunden vorzusehen. Die Ausbeuten betragen 90 % für Ausgangsmaterial Theophyllin und 85 % für Ausgangsmaterial Theophyllin-Ca.

### Beispiel 5

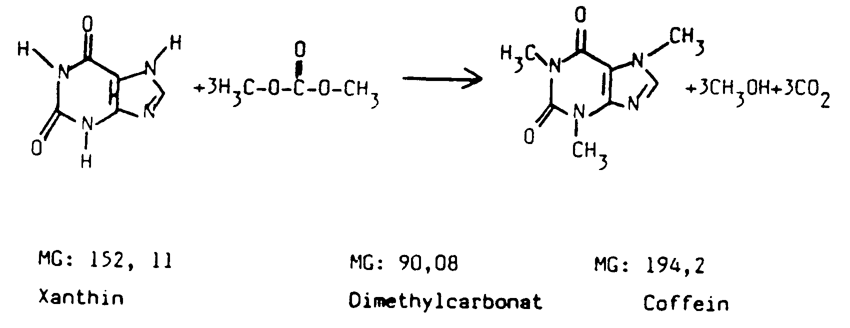

In einem 500 ml Schüttelautoklav werden 55 ml Methanol und 110 ml (1,3 Mol) Dimethylcarbonat vorgelegt und 20 g (0,13 Mol) Xanthin zugegeben. Man erhitzt die Suspension auf 200°C. Während des 5 stündigen Schüttelns steigt der Druck auf ca. 70 bar an. Nach Ende der Reaktionszeit wird auf ca. 70°C abgekühlt belüftet und Methanol bis zu einem Restvolumen von 100 ml abdestilliert. Die Suspension (∼pH 8,3) wird mittels Eisbad abgekühlt. Die Kristalle werden abgesaugt und mit 2x30 ml kaltem Methanol gewaschen. Die isolierte Substanz wird bei 50°C im Vakuumtrockenschrank bis zur Gewichtskonstanz getrocknet.
Ausbeute: 21,1 g (≙ 82,6 %)
Schmelzpunkt: 234-237°C
HPLC: 99,8 %

### Beispiel 6

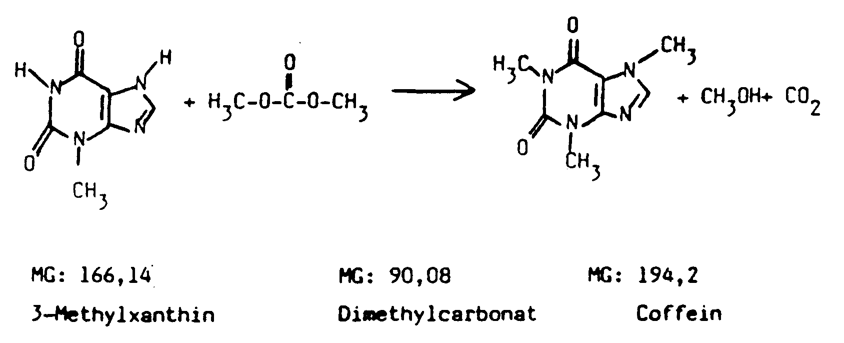

In einem 250 ml Schüttelautoklav werden 50 ml Dimethylcarbonat (0,6 Mol) und 25 ml Methanol vorgelegt. Dazu gibt man 10 g 3-Methylxanthin (0,06 Mol) und erhitzt die Suspension auf 200°C. Während des 5 stündigen Schüttelns steigt der Druck auf ca. 60 bar an.

Nach Ende der Reaktionszeit wird auf ca. 70°C abgekühlt, belüftet, in einen Erlenmeyerkolben überführt und weiter auf 10°C gekühlt. Die ausgefallenen Kristalle saugt man ab und wäscht mit 15 ml kaltem Methanol nach. Die isolierte Substanz wird bei 50°C im Vakuumtrockenschrank bis zur Gewichtskonstanz getrocknet.
Ausbeute: 9,3 g (≙ 79,6 %)
Fp : 234-237°C
HPLC : 99,4 %

## Patentansprüche

1. Verfahren zum Methylieren von Xanthinen, dadurch gekennzeichnet, daß Dimethylcarbonat als Methylierungsmittel verwendet wird.

2. Verfahren nach Anspruch 1, dadurch gekennzeichnet, daß das Molverhältnis von Ausgangsmaterial zu Dimethylcarbonat 1 : 3 bis 1 : 50 ist.

3. Verfahren nach Anspruch 1 oder 2, dadurch gekennzeichnet, daß die Reaktionsmischung mittels eines wäßrigen Puffers auf pH 7,8 bis 7,1 eingestellt wird.

4. Verfahren nach Anspruch 1 oder 2, dadurch gekennzeichnet, daß der Reaktionsmischung ein organisches Lösungsmittel mit acidem Wasserstoff, vorzugsweise Methanol, zugesetzt wird.

5. Verfahren nach einem der Ansprüche 1 bis 4, dadurch gekennzeichnet, daß die Umsetzung bei 100 bis 250°C unter entsprechend erhöhtem Druck durchgeführt wird.

6. Verfahren nach Anspruch 1, dadurch gekennzeichnet, daß Xanthin oder 3-Methylxanthin oder deren Natrium- oder Calciumsalz zu Theobromin methyliert wird, indem Dimethylcarbonat in einem Molverhältnis Ausgangsmaterial zu Dimethylcarbonat von 1 zu 3 bis 1 zu 50, vorzugsweise 1 zu 7 bis 1 zu 10, verwendet wird und die Umsetzung in Wasser oder wäßriger Pufferlösung (pH 7,8-7,1) bei 100 bis 250°C, vorzugsweise 170 bis 200°C, unter entsprechend erhöhtem Druck ausgeführt wird.

7. Verfahren nach Anspruch 1, dadurch gekennzeichnet, daß Xanthin, 3-Methylxanthin oder Theophyllin oder deren Natrium- oder Calciumsalz zu Coffein methyliert wird, indem Dimethylcarbonat in einem Molverhältnis Ausgangsmaterial zu Dimethylcarbonat von 1 zu 3 bis 1 zu 50, vorzugsweise 1 zu 7 bis 1 zu 10, verwendet wird und die Umsetzung bei 100 bis 250°C, vorzugsweise 150 bis 200°C unter entsprechend erhöhtem Druck ausgeführt wird.

8. Verfahren nach Anspruch 7, dadurch gekennzeichnet, daß ein acides Lösungsmittel der Reaktionsmischung zugefügt wird.

9. Verfahren nach Anspruch 8, dadurch gekennzeichnet, daß Methanol der Reaktionsmischung zugefügt wird.

10. Verfahren nach Anspruch 9, dadurch gekennzeichnet, daß Methanol in einem Volumsverhältnis Dimethylcarbonat zu Methanol von 3,3 zu 1 bis 2 zu 1 zugefügt wird.

## Claims

1. Process for methylating xanthines, characterised in that dimethylcarbonate is used as methylating agent.

2. Process as claimed in claim 1, characterised in that the molar ratio of starting material to dimethylcarbonate is 1:7 to 1:10.

3. Process as claimed in claim 1 or 2, characterised in that the reaction mixture is adjusted to pH 7.8 to 7.1 by means of an aqueous buffer.

4. Process as claimed in claim 1 or 2, characterised in that an organic solvent with acid hydrogen, preferably methanol, is added to the reaction mixture.

5. Process as claimed in one of claims 1 to 4, characterised in that the reaction is carried out at 100 to 200°C under correspondingly elevated pressure.

6. Process as claimed in claim 1, characterised in that xanthine or 3-methylxanthine or a sodium or calcium salt thereof is methylated to form theobromine, using dimethylcarbonate in a molar ratio of starting material to dimethylcarbonate of from 1:3 to 1:50, preferably 1:7 to 1:10 and the reaction being carried out in water or agueous buffer solution (pH 7.8-7.1) at 100 to 250°C, preferably at 170 to 200°C, under correspondingly elevated pressure.

7. Process as claimed in claim 1, characterised in that xanthine, 3-methylxanthine or theophylline or a sodium or calcium salt thereof is methylated to form caffeine, using dimethylcarbonate in a molar ratio of starting material to dimethylcarbonate of from 1:3 to 1:50, preferably 1:7 to 1:10, and the reaction being carried out at 100 to 250°C, preferably 150 to 200°C, under correspondingly elevated pressure.

8. Process as claimed in claim 7, characterised in that an acidic solvent is added to the reaction mixture.

9. Process as claimed in claim 8, characterised in that methanol is added to the reaction mixture.

10. Process as claimed in claim 9, characterised in that methanol is added in a volume ratio of dimethylcarbonate to methanol of from 3.3:1 to 2:1.

## Revendications

1. Procédé de méthylation de xanthines, caractérisé en ce que l'on utilise le carbonate de diméthyle comme agent de méthylation.

2. Procédé selon la revendication 1, caractérisé en ce que le rapport molaire du produit de départ au carbonate de diméthyle est de 1:3 à 1:50.

3. Procédé selon la revendication 1 ou 2, caractérisé en ce que le mélange réactionnel est amené à un pH de 7,8 à 7,1 au moyen d'un tampon aqueux.

4. Procédé selon la revendication 1 ou 2, caractérisé en ce qu'un solvant organique à hydrogène acide, de préférence le méthanol, est ajouté au mélange réactionnel.

5. Procédé selon l'une des revendications 1 à 4, caractérisé en ce que la réaction est accomplie entre 100 et 250°C sous une pression élevée correspondante.

6. Procédé selon la revendication 1, caractérisé en ce que l'on méthyle en théobromine la xanthine ou la 3-méthyl-xanthine ou leur sel de sodium ou de calcium en utilisant le carbonate de diméthyle dans un rapport molaire du produit de départ au carbonate de diméthyle de 1 à 3 à 1 à 50, de préférence de 1 à 7 à 1 à 10, et en conduisant la réaction dans l'eau ou dans une solution tampon aqueuse (pH 7,8-7,1) entre 100 et 250°C, de préférence entre 170 et 200°C, sous une pression élevée correspondante.

7. Procédé selon la revendication 1, caractérisé en ce que l'on méthyle en caféine la xanthine, la 3-méthylxanthine ou la théophylline ou leur sel de sodium ou de calcium en utilisant le carbonate de diméthyle dans un rapport molaire du produit de départ au carbonate de diméthyle de 1 à 3 à 1 à 50, de préférence de 1 à 7 à 1 à 10, et en conduisant la réaction entre 100 et 250°C, de préférence entre 150 et 200°C, sous une pression élevée correspondante.

8. Procédé selon la revendication 7, caractérisé en ce que l'on ajoute un solvant acide au mélange réactionnel.

9. Procédé selon la revendication 8, caractérisé en ce que l'on ajoute du méthanol au mélange réactionnel.

10. Procédé selon la revendication 9, caractérisé en ce que l'on ajoute le méthanol dans un rapport volumique du carbonate de diméthyle au méthanol de 3,3 à 1 à 2 à 1.
